(19)

Europäisches
Patentamt

European
Patent Office

Office européen
des brevets

(11) **EP 3 740 759 B1**

(12) **EUROPEAN PATENT SPECIFICATION**

(45) Date of publication and mention
of the grant of the patent:
**30.04.2025 Bulletin 2025/18**

(21) Application number: **19700162.1**

(22) Date of filing: **09.01.2019**

(51) International Patent Classification (IPC):
***G01N 33/68*** *(2006.01)*

(52) Cooperative Patent Classification (CPC):
**G01N 33/6857**; G01N 2800/18

(86) International application number:
**PCT/EP2019/050364**

(87) International publication number:
**WO 2019/141547 (25.07.2019 Gazette 2019/30)**

(54) **PERIODONTITIS DIAGNOSTIC METHODS, USES AND KITS**

METHODEN, VERWENDUNGEN UND KITS ZUR DIAGNOSE VON PERIODONTITIS

METHODES, USAGE ET KITS POUR LA DIAGNOSE DE LA PERIODONTITITS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.01.2018 EP 18151839**

(43) Date of publication of application:
**25.11.2020 Bulletin 2020/48**

(73) Proprietor: **Koninklijke Philips N.V.**
**5656 AG Eindhoven (NL)**

(72) Inventors:
• KOOIJMAN, Gerben
5656 AE Eindhoven (NL)
• RMAILE, Amir, Hussein
5656 AE Eindhoven (NL)
• GLASSE, Carl
5656 AE Eindhoven (NL)
• DE JAGER, Marinus, Karel, Johannes
5656 AE Eindhoven (NL)
• CHAPPLE, Iain, Leslie, Campbell
5656 AE Eindhoven (NL)
• GRANT, Melissa, Mackay
5656 AE Eindhoven (NL)
• PRESHAW, Philip
5656 AE Eindhoven (NL)
• TAYLOR, John, J
5656 AE Eindhoven (NL)
• VAN HARTSKAMP, Michael, Alex
5656 AE Eindhoven (NL)

(74) Representative: **Philips Intellectual Property &
Standards**
**High Tech Campus 52**
**5656 AG Eindhoven (NL)**

(56) References cited:
**WO-A2-2014/037924     US-A1- 2006 160 233**

• **CARLO BERTOLDI ET AL: "Non-bacterial protein
expression in periodontal pockets by proteome
analysis", JOURNAL OF CLINICAL
PERIODONTOLOGY, vol. 40, no. 6, 1 June 2013
(2013-06-01), DK, pages 573 - 582, XP055454955,
ISSN: 0303-6979, DOI: 10.1111/jcpe.12050**
• **CHIARA BALDINI ET AL: "Proteomic analysis of
saliva: a unique tool to distinguish primary
SjÃgren's syndrome from secondary SjÃgren's
syndrome and other sicca syndromes",
ARTHRITIS RESEARCH AND THERAPY, BIOMED
CENTRAL, LONDON, GB, vol. 13, no. 6, 25
November 2011 (2011-11-25), pages R194,
XP021094475, ISSN: 1478-6354, DOI: 10.1186/
AR3523**
• **RAUBENHEIMER E ET AL: "Salivary
immunoglobulin related proteins in 24 patients
with multiple myeloma", EUROPEAN JOURNAL
OF CANCER. PART B, ORAL ONCOLOGY,
PERGAMON, OXFORD, GB, vol. 29, no. 4, 1
January 1993 (1993-01-01), pages 295 - 297,
XP022645576, ISSN: 0964-1955, [retrieved on
19930101], DOI: 10.1016/0964-1955(93)90051-F**

**(Cont. next page)**

EP 3 740 759 B1

- BASILE UMBERTO ET AL: "Free light chains: Eclectic multipurpose biomarker", JOURNAL OF IMMUNOLOGICAL METHODS, vol. 451, 18 September 2017 (2017-09-18), pages 11 - 19, XP085287882, ISSN: 0022-1759, DOI: 10.1016/ J.JIM.2017.09.005
- JENNIFER L J HEANEY: "Salivary immunoglobulin free light chains: reference ranges and responses to exercise in young and older adults", EXERC IMMUNOL REV, vol. 22, 2016, pages 28 - 41, XP055568179, Retrieved from the Internet <URL:http://eir-isei.de/2016/ eir-2016-028-article.pdf> [retrieved on 20190312]
- Y. WU ET AL: "Effect of Aging on Periodontal Inflammation, Microbial Colonization, and Disease Susceptibility", JOURNAL OF DENTAL RESEARCH, vol. 95, no. 4, 1 April 2016 (2016-04-01), US, pages 460 - 466, XP055568250, ISSN: 0022-0345, DOI: 10.1177/ 0022034515625962
- JOHN J. TAYLOR: "Protein Biomarkers of Periodontitis in Saliva", ISRN INFLAMMATION, vol. 141, no. 1, 1 January 2014 (2014-01-01), pages 52 - 18, XP055131500, DOI: 10.1155/2014/ 593151

**Description**

FIELD OF THE INVENTION

The invention is in the field of oral care, and pertains to saliva-based diagnostics of periodontal disease. Particularly, the invention pertains to a system, use and method for diagnosing periodontitis.

BACKGROUND OF THE INVENTION

Gum inflammation, or gingivitis, is a non-destructive periodontal disease caused mainly by the adherence of dental bacterial biofilms, or dental plaque, to the tooth surface. If not detected and treated, the reversible gingivitis usually leads to the inflammation of the tissues surrounding the tooth (i.e. periodontal tissues), a condition defined as periodontitis, which is irreversible and causes tissue destruction and alveolar bone loss, and ultimately results in the loss of teeth. During the progression of gum disease, there are usually clinical signs and symptoms associated with it, such as the swelling of the gums, the change in color from pink to dark red, the bleeding of the gums, bad breath, and the gums becoming more tender or painful to touch.

Periodontitis is a chronic multifactorial inflammatory disease caused by oral microorganisms and characterized by progressive destruction of the hard (bone) and soft (periodontal ligament) tissues, ultimately leading to tooth mobility and loss. This is to be distinguished from gingivitis which is a reversible infection and inflammation of the gum tissues. Inflammatory periodontitis is one of the most prevalent chronic human diseases and a major cause of adult tooth loss. In addition to the substantial negative impact of periodontitis on oral health, there is also mounting evidence that periodontitis has systemic consequences and that it is a risk factor for several systemic diseases, including heart diseases (e.g. atherosclerosis, stroke), diabetes, pregnancy complications, rheumatoid arthritis and respiratory infections.

Early and accurate diagnosis of periodontal disease, thus, is important from both an oral and overall health perspective.

Periodontal diseases are still poorly diagnosed in general dental practice, resulting in relatively low rates of therapeutic intervention and significant amounts of untreated cases. Current diagnosis relies on imprecise, subjective clinical examination of oral tissue condition (color, swelling, extent of bleeding on probing, probing pocket depth; and bone loss from oral x-rays) by dental professionals. These conventional methods are time consuming, and some of the techniques used (pocket-depth, x-ray) reflect historic events, such as past disease activity, rather than current disease activity or susceptibility to further disease. Hence, more objective, faster, accurate, easier-to-use diagnostics which preferably may also be performed by non-specialists are desirable. Thereby it is desirable to measure current disease activity, and possibly a subject's susceptibility to further periodontal disease.

Saliva or oral fluids have long been advocated as a diagnostic fluid for oral and general diseases, and with the advent of miniaturized biosensors, also referred to as lab-on-a-chip, point of care diagnostics for rapid chair-side testing have gained greater scientific and clinical interest. Especially for periodontal disease detection, inflammatory biomarkers associated with tissue inflammation and breakdown may easily end up in saliva due to proximity, suggesting saliva has strong potential for periodontal disease detection. Indeed, this area thus has gained significant interest and encouraging results have been presented. For example, WO2014-A-037924 describes methods for diagnosing the status of periodontitis disease including selecting a set of protein biomarkers including one or more biomarkers which have been shown to vary in abundance at particular stages of periodontitis. Ramseier et al (J Periodontol. 2009 Mar;80(3):436-46) identified host- and bacterially derived biomarkers correlated with periodontal disease. However, no definite test has emerged yet.

Biomarkers represent biological indicators that underpin clinical manifestations, and as such are objective measures by which to diagnose clinical outcomes of periodontal disease. Ultimately, proven biomarkers could be utilized to assess risk for future disease, to identify disease at the very earliest stages, to identify response to initial therapy, and to allow implementation of preventive strategies.

Previous limitations to the development of point-of-care tests for salivary biomarkers included a lack of technologies that were adaptable to chair-side applications and an inability to analyze multiple biomarkers in individual samples. Also the selection of which multiple biomarkers to include in such a test has not been adequately addressed in the literature nor implemented in practical tests.

It would be desired to provide a simpler process, and particularly a process that requires only that a small saliva sample is taken from a patient, and possibly by the patient him- or herself. It is desired that such a sample be entered into an *in vitro* diagnostic device, which will allow, based on measurement, a classification of the saliva sample such that it can return an indication of the likelihood that the patient is to be classified as suffering from periodontitis.

SUMMARY OF THE INVENTION

In order to better address the foregoing desires, the invention, in one aspect, concerns an *in vitro* method for assessing whether a human patient has periodontitis, the method comprising detecting, in a sample of saliva from said human patient, the concentrations of the Free Light Chain κ protein and/or the Free Light Chain λ protein; determining a testing value reflecting the concentration or joint concentrations determined for said protein or proteins; comparing the testing value with a threshold value reflecting in the same manner the concentration or joint concentrations associated with periodontitis, so as to assess whether the testing value is indicative for periodontitis in said patient.

In another aspect, the invention presents the use of the Free Light Chain κ protein and/or the Free Light Chain λ in a saliva sample of a human patient, as biomarkers for assessing whether the patient has periodontitis.

Optionally, the age of the patient is also used as a biomarker.

In a further aspect, the invention resides in a system for assessing whether a human patient has periodontitis, the system comprising:

- detection means able and adapted to detect in a sample of saliva of the human patient the Free Light Chain κ protein and/or the Free Light Chain λ; and
- a processor able and adapted to determine from the determined concentrations of said proteins an indication of the patient having periodontitis.

The system optionally contains a data connection to an interface, particularly a graphical user interface, capable of presenting information, preferably also capable of putting in information, said interface being either a part of the system or a remote interface.

Optionally one or more of the foregoing items, particularly the processor, are enabled to function "in the cloud", i.e., not on a fixed machine, but by means of an internet-based application.

In yet another aspect, the invention provides an *in vitro* method for determining a change in status of periodontitis in a human patient over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the Free Light Chain λ protein and/or the Free Light Chain κ protein, and comparing the concentrations, whereby a difference in either or both of the concentrations, reflects a change in status.

In a further aspect, the invention provides a method of diagnosing whether a human patient has periodontitis, comprising detecting in a sample of saliva of the human patient the Free Light Chain λ protein and/or the Free Light Chain κ protein, and assessing the presence of periodontitis in the patient on the basis of the concentrations of said protein or proteins in said sample. Optionally, the method of this aspect comprises the further step of treating the periodontitis in the patient.

BRIEF DESCRIPTION OF THE DRAWINGS

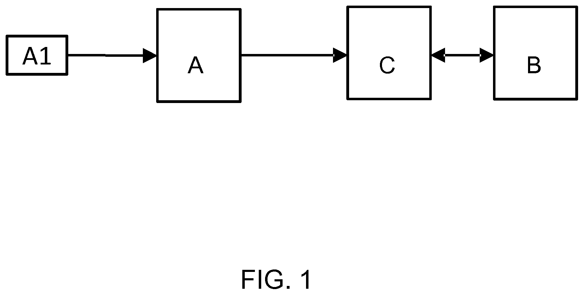

Fig. 1 schematically represents a system for use in the method as described in this disclosure.

DETAILED DESCRIPTION OF THE INVENTION

In a general sense, the invention is based on the judicious insight that even a single protein can serve as a biomarker in a sample of saliva of a human patient, for identifying the presence or absence of periodontitis. Two proteins have been identified that can be used alone or in combination to diagnose periodontitis.

These proteins are the Free Light Chain λ protein and/or the Free Light Chain κ protein. When a single biomarker protein is used, Free Light Chain λ is preferred.

When both proteins are detected, the sum of, ratio of, or difference between, the two proteins can be determined in certain embodiments. The sum, ratio and/or difference can optionally be used in combination with one another. For example, the ratio and sum of the two chains may be combined according to one embodiment, or the ratio and the difference may be combined in another embodiment. The sum, ratio, difference or combination thereof can optionally be further combined with the measures of Free Light Chain λ protein and/or the Free Light Chain κ protein. For example: the κ/λ ratio may be usefully combined with the measure of Free Light Chain λ; the κ/λ ratio may be usefully combined with the measure of Free Light Chain κ; or the κ/λ ratio may be usefully combined with the measure of Free Light Chain κ and Free Light Chain λ.

The subject's age may optionally be included as an additional marker.

Free Light Chain proteins are immunoglobulin light chains. They are not associated with an immunoglobulin heavy chain. Unlike a typical whole immunoglobulin molecule, a Free Light Chain protein is not covalently linked to an immunoglobulin heavy chain, e.g. the Free Light Chain is not disulphide bonded to a heavy chain. Typically the Free Light Chain comprises approximately 220 amino acids. Typically, the Free Light Chain protein comprises a variable region (often referred to as the Light Chain variable Region, $V_L$) and a constant region (often referred to as the Light Chain constant Region, $C_L$). Humans produce two types of immunoglobulin light chains, named with the letter kappa (κ) and lambda (λ).

Each of these can be further divided into sub-groups based on variation in the variable region, with four kappa subtypes (V$\kappa$1, V$\kappa$2, V$\kappa$3 and V$\kappa$4) and six lambda subtypes (V$\lambda$1, V$\lambda$2, V$\lambda$3, V$\lambda$4, V$\lambda$5 and V$\lambda$6). Free Light Chain $\kappa$ is typically monomeric. Free Light Chain $\lambda$ is typically dimeric, linked by disulphide bonding (to another Free Light Chain $\lambda$). Polymeric forms of Free Light Chain $\lambda$ and of Free Light Chain $\kappa$ have been identified. Free light chains are produced by bone marrow and lymph node cells as well as locally in the periodontium by diffuse lymphocytes, and are rapidly cleared from the blood and catabolised by the kidneys. Monomeric free light chains are cleared in 2-4 hours, and dimeric free light chains in 3-6 hours.

The two proteins mentioned above are known in the art. The skilled person is aware of their structure, and of methods to detect them in an aqueous sample, such as a saliva sample. Hereinafter the aforementioned protein biomarkers are collectively referred to as "the biomarker panels of the invention." A biomarker panel of the invention, in one embodiment, consists of the two protein biomarkers identified in the invention, i.e., Free Light Chain $\lambda$ and Free Light Chain $\kappa$. In addition to the biomarker panels of the invention, other biomarkers and or data, such as demographic data (e.g., age, sex) can be included in a set of data applied for the determination of the type of periodontitis.

When other biomarkers are optionally included, the total number of biomarkers (i.e. the biomarker panel of the invention plus other biomarkers) is typically 3, 4, 5 or 6.

However, a desirable advantage of the present invention is that the classification of periodontitis in a patient can be determined by measuring preferably not more than two biomarkers, with the biomarker panel of both Free Light Chain $\lambda$ and Free Light Chain $\kappa$ being preferred. Particularly, the determination does not need to involve the use of other data, which advantageously provides a simple and straightforward diagnostic test.

The method, as desired, requires only that a small saliva sample, e.g. a dropsize, is taken from the subject. The size of the sample will typically range of from 0.1 $\mu$l to 2 ml, such as 1-2 ml, whereby smaller amounts, e.g., 0.1 to 100 $\mu$l can be used for *in vitro* device processing, and whereby taking a larger sample, such as up to 20 ml, such as 7.5 to 17 ml, is also possible.

This sample is entered into an *in vitro* diagnostic device, which measures the concentration(s) of the protein or proteins involved, and which returns a diagnostic outcome, classifying the subject on the basis of a likelihood of having periodontitis.

The ease of use of this invention will make it possible to test the majority of dental patients with periodontitis, or with a high risk for developing periodontitis, on a regular basis (e.g. as part of a regular dental check or even at home). This allows, *inter alia,* detecting the presence of periodontitis soon after it has developed, and thus enables more timely taking oral care measures to prevent periodontitis from advancing. Or, e.g., with patients known to be at high risk for periodontitis, and tested for the first time, the method allows to identify whether the periodontitis has developed. Also, the method can be applied after treatment of a patient previously diagnosed with periodontitis, in order to check whether the periodontitis has been successfully treated. Particularly, the method is also suitable for self-diagnosis, whereby the steps of taking the sample and entering it into a device can be conducted by the patient him- or herself.

The patient may typically be known or suspected to have periodontitis when the invention is carried out to confirm whether the periodontitis is present. In certain embodiments therefore, the method is for assessing whether a human patient, known or suspected to have periodontitis, has periodontitis.

A method of the invention typically comprises detecting the aforementioned protein or proteins making up a biomarker panel of the invention, and optional further biomarker proteins, by using one or more detection reagents.

The "saliva" that is tested according to the invention may be undiluted saliva, which may be obtained by spitting or swabbing, or diluted saliva, which may be obtained by rinsing the mouth with a fluid. Diluted saliva may be obtained by the patient rinsing or swilling their mouth for a few seconds with sterile water (for example 5ml or 10ml) or other suitable fluid, and spitting into a container. Diluted saliva may sometimes be referred to as an oral rinse fluid.

By "detecting" is meant measuring, quantifying, scoring, or assaying the concentration of the biomarker proteins. Methods of evaluating biological compounds, including biomarker proteins, are known in the art. It is recognized that methods of detecting a protein biomarker include direct measurements and indirect measurements. One skilled in the art will be able to select an appropriate method of assaying a particular biomarker protein.

The term "concentration" with respect to the protein biomarkers is to be given its usual meaning, namely the abundance of the protein in a volume. Protein concentration is typically measured in mass per volume, most typically mg/ml, $\mu$g/ml or ng/ml, but sometimes as low as pg/ml. An alternative measure is Molarity (or Molar concentration), mol/L or "M". The concentration can be determined by detecting the amount of protein in a sample of known, determined or pre-determined volume.

An alternative to determining the concentration is to determine the absolute amount of the protein biomarker in the sample, or determining the mass-fraction of the biomarker in the sample, for example the amount of the biomarker relative to the total of all other proteins in the sample.

A "detection reagent" is an agent or compound that specifically (or selectively) binds to, interacts with or detects the protein biomarker of interest. Such detection reagents may include, but are not limited to, an antibody, polyclonal antibody, or monoclonal antibody that preferentially binds the protein biomarker.

The phrase "specifically (or selectively) binds" or "specifically (or selectively) immunoreactive with," when referring to a detection reagent, refers to a binding reaction that is determinative of the presence of the protein biomarker in a heterogeneous population of proteins and other biologics. Thus, under designated immunoassay conditions, the specified detection reagent (e.g. antibody) binds to a particular protein at least two times the background and does not substantially bind in a significant amount to other proteins present in the sample. Specific binding under such conditions may require an antibody that is selected for its specificity for a particular protein. A variety of immunoassay formats may be used to select antibodies specifically immunoreactive with a particular protein. For example, solid-phase ELISA immunoassays (enzyme linked immunosorbent assay) are routinely used to select antibodies specifically immunoreactive with a protein (*see, e.g.,* Harlow & Lane, Antibodies, A Laboratory Manual (1988), for a description of immunoassay formats and conditions that can be used to determine specific immunoreactivity). Typically a specific or selective reaction will be at least twice the background signal or noise and more typically more than 10 to 100 times the background.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region. The antibody may be a bispecific antibody, e.g. an antibody that has a first variable region that specifically binds to a first antigen and a second variable region that specifically binds to a second, different, antigen. Use of at least one bispecific antibody can reduce the number of detection reagents needed.

Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive.

The biomarker protein(s) of the invention can be detected in a sample by any means. Preferred methods for biomarker detection are antibody-based assays, protein array assays, mass spectrometry (MS) based assays, and (near) infrared spectroscopy based assays. For example, immunoassays, include but are not limited to competitive and non-competitive assay systems using techniques such as Western blots, radioimmunoassays, ELISA, "sandwich" immunoassays, immunoprecipitation assays, precipitin reactions, gel diffusion precipitin reactions, immunodiffusion assays, fluorescent immunoassays and the like. Such assays are routine and well known in the art. Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1 % sodium deoxycholate, 0.1% SDS, 0.15 M NaC1, 0.01 M sodium phosphate at pH 7.2, 1 % Trasylol) supplemented with protein phosphatase and/or protease inhibitors (e.g., EDTA, PMSF, aprotinin, sodium vanadate), adding an antibody of interest to the cell lysate, incubating for a period of time (e.g., 1-4 hours) at 4°C, adding protein A and/or protein G sepharose beads to the cell lysate, incubating for about an hour or more at 4°C, washing the beads in lysis buffer and resuspending the beads in SDS/sample buffer. The ability of the antibody to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with Sepharose beads).

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 125I) diluted in blocking buffer, washing the membrane in wash buffer, and detecting the presence of the antigen. One of skill in the art would be knowledgeable as to the parameters that can be modified to increase the signal detected and to reduce the background noise.

ELISAs typically comprise preparing antigen (i.e. the biomarker protein of interest or fragment thereof), coating the well of a 96- well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art.

When multiple markers are used, a threshold is determined on the basis of the joint concentrations of these biomarkers. When a single biomarker is used, a threshold is determined on the basis of the concentration of that biomarker. The threshold determines whether a patient is classified as having periodontitis or not. The invention reflects the insight that periodontitis can be detected, with sufficient accuracy based on a measurement of the single biomarker or combination of two biomarkers as indicated above.

This insight supports another aspect, the invention, which is the use of the Free Light Chain $\lambda$ protein and/or Free Light Chain $\kappa$ protein, as a biomarker or biomarkers in a saliva sample of a human patient, for assessing whether the patient has periodontitis.

This use can be implemented in a method as substantially described hereinbefore and hereinafter.

When both Free Light Chain $\lambda$ and Free Light Chain $\kappa$ are detected, the method of the invention comprises determining a testing value reflecting the joint concentrations measured for said proteins. A joint concentration value can be any value obtained by input of the concentrations as determined and an arithmetic operation of these values. This can, e.g., be a simple addition of the concentrations. It can also involve multiplying each concentration with a factor reflecting a desired weight of these concentrations, and then adding up the results. It can also involve multiplying the concentrations with each other, or any combination of multiplication, division, subtraction, exponentiation, and addition. It can further involve raising concentrations to some power. In preferred embodiments, the sum of the two biomarkers is used ($\kappa+\lambda$). In other preferred embodiments, the ratio of the two biomarkers is used (e.g. $\kappa/\lambda$ or $\lambda/\kappa$). In other embodiments, the difference between the two biomarkers is used (e.g. $\kappa-\lambda$ or $\lambda-\kappa$).

Optionally, the testing value reflects the concentration of joint concentrations determined for said protein(s) in combination with the age of the subject.

The resulting joint concentration value is compared with a threshold value reflecting in the same manner the joint concentrations associated with the presence of periodontitis. The comparison allows assessing whether the testing value is indicative of the presence of periodontitis in the patients whose saliva is subjected to the test.

The threshold value can, e.g., be the concentration or joint concentration value, obtained in the same manner on the basis of the concentration(s) determined for the same protein(s) in a reference sample associated with the presence of periodontitis, i.e. in a patient diagnosed with periodontitis. Typically, thereby a value reflecting the same or higher joint concentration is indicative of the presence of periodontitis in a tested patient. Analogously, a value reflecting a lower joint concentration in the saliva of a tested periodontitis patient, indicates that periodontitis is absent. However, it will be understood that it is also possible to calculate a threshold value (e.g. by using a negative multiplier) such that a testing value indicating periodontitis would be below the threshold, and a testing value indicating absence of periodontitis, would be above the threshold.

The threshold value can also be determined on the basis of measuring the concentration(s) of the present biomarker protein(s) in a set of samples, including patients with a known diagnosis of periodontitis and "not" periodontitis. Thereby the measured concentration values can be subjected to statistical analysis, possibly including machine learning methods, allowing to discriminate, with the desired sensitivity and specificity, patients classified as periodontitis and patients classified as not suffering from periodontitis. Therefrom, the desired threshold value can be obtained. On the basis of this threshold value, a sample to be tested can be subjected to the same concentration measurement, and the concentration values are then processed, in the same manner in which the threshold value is obtained, so as to determine a joint concentration value that can be compared with the threshold, thus allowing the tested sample to be classified as having periodontitis or not.

In an interesting embodiment, the concentration or joint concentration value is obtained in the form of a score as follows. A numerical value (protein concentration values in e.g. ng/ml) is assigned to each measurement, and these values are used in a linear or nonlinear combination to calculate a score between zero and one. In the event that the threshold value is determined on the basis of a set of subjects as mentioned above, the score between 0 and 1 is typically calculated with the sigmoid function that takes the joint concentration as input (as shown further on).

When the score exceeds a certain threshold, the method indicates that the patient has periodontitis. The threshold may be chosen based on the desired sensitivity and specificity.

It will be understood that in performing a 'periodontitis classification' on a subject, in accordance with the invention, this is on subjects that can be assumed to be at risk from, or suffering from, periodontitis. This can either be known from e.g. a previously performed diagnosis of periodontitis, though perhaps without ability to differentiate the extent of it, or, e.g., assumed from the subject's oral health condition record.

Clinical definitions as acknowledged in the art are based on the following:

*Gingival Index (GI)*

A full mouth gingival index will be recorded based on the Lobene Modified Gingival Index (MGI) rated on a scale of 0 to 4, where:

- 0 = absence of inflammation,
- 1 = mild inflammation; slight change in color little change in texture of any portion of but not the entire margin or papillary gingival unit,
- 2 = mild inflammation; but involving entire margin or papillary unit,
- 3 = moderate inflammation; glazing, redness, oedema and/or hypertrophy of margin or papillary unit,
- 4 = severe inflammation; marked redness, oedema and/or hypertrophy of marginal or papillary gingival unit, spontaneous bleeding, congestion, or ulceration].

*Probing depths (PD)*

Probing depths will be recorded to the nearest mm using a manual UNC-15 periodontal probe. Probing depth is the distance from the probe tip (assumed to be at the base of the pocket) to the free gingival margin.

*Gingival recession (REC)*

Gingival recession will be recorded to the nearest mm using a manual UNC-15 periodontal probe. Gingival recession is the distance from the free gingival margin to the cemento-enamel junction. Gingival recession will be indicated as a positive number and gingival overgrowth will be indicated as a negative number.

*Clinical attachment loss (CAL)*

Clinical attachment loss will be calculated as the sum of probing depth + recession at each site.

*Bleeding on probing (BOP)*

Following probing, each site will be assessed for bleeding on probing, if bleeding occurs within 30s of probing, a score of 1 will be assigned for the site, otherwise a score of 0 will be assigned.

The resulting subject group (patient group) definition is as follows, whereby the mild-moderate periodontitis and the advanced periodontitis groups are "periodontitis" relevant to the present invention:

- Healthy group (H): $PD \leq 3$ mm in all sites (but would allow up to four 4 mm pockets at distal of last standing molars), no sites with interproximal attachment loss, GI of $\geq 2.0$ in $\leq 10\%$ sites, %BOP scores $\leq 10\%$;
- Gingivitis group (G): $GI \geq 3.0$ in $> 30\%$ of sites, no sites with interproximal attachment loss, no sites with $PD > 4$ mm , % BOP scores $> 10\%$;
- Mild-moderate periodontitis group (MP): interproximal PD of 5-7 mm, (equating to approximately 2-4 mm CAL) at $\geq 8$ teeth, %BOP scores $> 30\%$;
- Advanced periodontitis group (AP): interproximal PD of $\geq 7$ mm, (equating to approximately $\geq 5$ mm CAL) at $\geq 12$ teeth, %BOP scores $> 30\%$.

In an embodiment, the method of the invention makes use of a system as represented schematically in Fig. 1. The system can be a single apparatus having various device components (units) integrated therein. The system can also have its various components, or some of these components, as separate apparatuses. The components shown in Fig. 1 are a measurement device (A), a graphical user interface (B) and a computer processing unit (C).

As mentioned above, the system of the invention comprises a data connection to an interface, whereby the interface itself can be a part of the system or can be a remote interface. The latter refers to the possibility to use a different apparatus, preferably a handheld apparatus such as a smartphone or a tablet computer, for providing the actual interface. The data connection in such cases will preferably involve wireless data transfer such as by Wi-Fi or Bluetooth, or by other techniques or standards.

The measurement device (A) is configured to receive a saliva sample, for example by putting a drop of saliva on a cartridge (A1), which can be inserted into the device (A). The device can be an existing device that is capable to determine, from the same saliva sample, the concentrations of at least one of the Free Light Chain λ and Free Light Chain κ proteins.

The measurement device (A) should be able to receive a saliva sample, for example by putting a drop of saliva on a cartridge (A1), which can be inserted into the device (A). The device may be an existing device that is capable to determine, from the same saliva sample, the concentrations of at least one of the Free Light Chain λ and Free Light Chain κ proteins.

The processing unit (C) receives numerical values for the protein concentrations from part (A). The unit (C) is provided with software (typically embedded software) allowing it to calculate a score (S) between 0 and 1. The software further includes a numerical value for the threshold (T). If the calculated value (S) exceeds (T), unit (C) will output an indication (I) of 'periodontitis' to the GUI (B), otherwise it will output 'no periodontitis'. A further embodiment may use the specific value of (S) to indicate the certainty with which the indication (I) is made. This can be a probability score, whereby 0.5 is a possible threshold value, and e.g. a score S=0.8 would indicate the probability of periodontitis. Interesting options are:

> Based on the score S, one can directly indicate a certainty, i.e. S=0.8 means 80% certainty of periodontitis; or
> To make the indication through the definition of a range R1-R2, such that when R1<S<R2, the indication (I) will read 'inconclusive'.

A specific calculation of the score can be implemented, e.g., by means of a sigmoid function applying the following formula:

$$S = \frac{1}{1 + \exp(-(c_0 + \sum_{i=1}^{N} c_i B_i))}$$

Wherein $N$ is the number of proteins/biomarkers used. $c_0$, $c_1$, etc. are coefficients (numerical values) and $B_1, B_2$, etc. are the respective protein concentrations.

Determining of the coefficients can be done by a training procedure:

- Select N1 subjects with periodontitis (as identified by a dentist via the current criteria) and N2 subjects without periodontitis (having healthy gums or gingivitis).
- Take a saliva sample from each subject and determine the protein concentrations of a combination of biomarkers as explained above.
- Define the score S to be 1 for periodontitis, and 0 for no periodontitis.
- Fit the sigmoid function to the scores and protein concentration values.

Other regression or machine learning methods (linear regression, neural network, support vector machine) may be used where the score S, is high for periodontitis patients and low for the non-periodontitis controls.

With reference to the aforementioned system, the invention also provides, in a further aspect, a system for assessing whether a human patient has periodontitis, the system comprising:

- detection means able and adapted to detect in a sample of saliva of the human patient the Free Light Chain κ protein and/or the Free Light Chain λ protein; As explained above, such means are known, and easily accessible to the skilled person; Typically, there is provided a container for receiving an oral sample of a subject therein, the container provided with the detection means;
- a processor able and adapted to determine from the determined concentrations of said proteins an indication of the patient having periodontitis.

  - Optionally, the system comprises a user interface (or a data connection to remote interface), particularly a graphical user interface (GUI), capable of presenting information; a GUI is a type of user interface that allows users to interact with electronic devices through graphical icons and visual indicators such as secondary notation, instead of text-based user interfaces, typed command labels or text navigation (none of such interface types being excluded in the present invention); GUIs are generally known, and are used typically in handheld mobile devices such as MP3 players, portable media players, gaming devices, smartphones and smaller household, office and industrial controls; as said, the interface optionally can also be chosen so as to be capable of putting in information, such as, e.g., the age of the subject, sex, BMI (Body Mass Index).

In an interesting aspect, the recognition of a biomarker panel of the invention is applied in monitoring the status of periodontitis in a human patient, over time. Accordingly, the invention also provides an *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$

and in at least one sample of saliva obtained from said patient at $t_2$, the concentrations of the Free Light Chain $\kappa$ protein and/or the Free Light Chain $\lambda$ protein, and comparing the concentrations, whereby a difference of preferably at least one, and more preferably both concentrations, reflects a change in status. This difference can be reviewed as a difference in concentrations, thus allowing a direct comparison without first generating a number between 0 and 1, or any other classification. It will be understood that the measurements received at both points in time can also be processed in just the same manner as done when determining the periodontitis status as above.

The invention also provides a method of diagnosing whether a human patient has periodontitis, comprising detecting in a sample of saliva of the human patient the Free Light Chain $\kappa$ protein and/or the Free Light Chain $\lambda$ protein. The presence of periodontitis in the patient is typically assessed on the basis of the concentrations of said proteins in said sample. Optionally, the method of this aspect comprises the further step of treating the periodontitis in the patient. This optional treatment step can comprise the administration of known therapeutic agents or dental procedures, or a combination of therapeutic agents and dental procedures. Known therapeutic agents include the administration of antimicrobial-containing agents such as a mouthwash, chip, gel or microsphere. A typical antimicrobial agent for use in treating periodontitis is chlorhexidine. Other therapeutic agents include antibiotics, typically orally-administered antibiotics, and enzyme suppressants such as doxycycline. Known non-surgical therapeutic procedures include scaling and root planing (SRP). Known surgical procedures include surgical pocket reduction, flap surgery, gum grafts or bone grafts.

The invention will be further illustrated with reference to the following nonlimiting example.

Example

A clinical study was carried out with 153 subjects with varying periodontal health statuses (identified by clinical assessment by a dental professional via current criteria, e.g. American Academy of Periodontology criteria). 39 of the subjects had healthy gums (H), 35 were diagnosed with gingivitis (G), 41 were diagnosed with mild periodontitis (MP) and 38 with advanced periodontitis (AP), ROC (Receiver-Operator-Characteristic) Area-Under-the Curve (AUC) values were obtained following repeated clinical visits at two independent clinical sites.

Performance of various biomarker combinations were evaluated by means of logistic regression with leave-one-out cross validation (LOOCV), resulting in the preferred biomarker combinations as explained herein.

In statistics, a receiver operating characteristic curve, or ROC curve, is a graphical plot that illustrates the performance of a binary classifier system as its discrimination threshold is varied. The curve is created by plotting the true positive rate (TPR) against the false positive rate (FPR) at various threshold settings. The true-positive rate is also known as sensitivity, recall or *probability of detection* in machine learning. The false-positive rate is also known as the fall-out or *probability of false alarm* and can be calculated as (1 - specificity). The ROC curve is thus the sensitivity as a function of fall-out. In general, if the probability distributions for both detection and false alarm are known, the ROC curve can be generated by plotting the cumulative distribution function (area under the probability distribution from - $\infty$ to the discrimination threshold) of the detection probability on the y-axis, versus the cumulative distribution function of the false-alarm probability on the x-axis. The accuracy of the test depends on how well the test separates the group being tested into those with and without the disease in question. Accuracy is measured by the area under the ROC curve. An area of 1 represents a perfect test; an area of 0.5 represents a worthless test. A guide for classifying the accuracy of a diagnostic test is the traditional academic point system:

- 0.90-1 = excellent (A)
- 0.80-0.90 = good (B)
- 0.70-0.80 = fair (C)
- 0.60-0.70 = poor (D)
- 0.50-0.60 = fail (F)

Based on the foregoing, in the results of the aforementioned clinical study, an ROC AUC value of above 0.75 is considered to represent a desirable accuracy for providing a diagnostic test in accordance with the invention.

Table 1 below indicates that Receiver-Operator-Characteristic Area-Under-the Curve values of >0.8 for detecting periodontitis, were obtained using the claimed panels biomarkers.

The biomarker proteins presented in the table are FLC $\kappa$, FLC $\lambda$, the ratio $\kappa / \lambda$, the sum $\kappa + \lambda$, and the difference $\kappa - \lambda$.

In the table, it can be seen that maximum results, in terms of ROC AUC of 0.847 are obtained with biomarker panel consisting of the ratio $\kappa / \lambda$, the sum $\kappa + \lambda$, and age. A result close to this, at 0.835, is obtained with FLC $\kappa$, FLC $\lambda$, the ratio $\kappa / \lambda$ and age. 0.835 is also obtained with FLC $\lambda$, the ratio $\kappa / \lambda$ and age. Scores above 0.8 are reported for a number of other markers, including FLC $\lambda$ alone.

Table 1

| Age | Kappa | Lambda | KL sum | KL ratio | KL diff | AUC LOOCV |
|---|---|---|---|---|---|---|
| Single marker (plus optionally age) | | | | | | |
|  | X |  |  |  |  | 0.688 |
| X | X |  |  |  |  | 0.794 |
|  |  | X |  |  |  | 0.807 |
| X |  | X |  |  |  | 0.838 |
| Both $\kappa$ and $\lambda$ (plus optionally age) | | | | | | |
|  | X | X |  |  |  | 0.805 |
| X | X | X |  |  |  | 0.830 |
|  | X |  |  | X |  | 0.810 |
| X | X |  |  | X |  | 0.850 |
|  |  | X |  | X |  | 0.802 |
| X |  | X |  | X |  | 0.835 |
|  |  |  |  | X |  | 0.558 |
| X |  |  |  | X |  | 0.807 |
|  | X | X |  | X |  | 0.805 |
| X | X | X |  | X |  | 0.835 |
|  |  |  | X |  |  | 0.752 |
| X |  |  | X |  |  | 0.821 |
|  |  |  |  |  | X | 0.522 |
| X |  |  |  |  | X | 0.769 |
|  |  |  | X | X |  | 0.810 |
| X |  |  | X | X |  | 0.847 |
|  |  |  |  | X | X | 0.792 |
| X |  |  |  | X | X | 0.825 |

This table shows the performance of FLC biomarker panels, with optionally age added as marker, for classifying periodontitis versus non-periodontitis by means of logistic regression.

Next to the markers $\kappa$ and $\lambda$, additional predictors $\kappa+\lambda$, $\kappa-\lambda$, K/$\lambda$ were considered. Redundant panels are not shown in the table. The term redundant here reflects the fact that a panel including e.g. $\kappa+\lambda$ and $\kappa-\lambda$ as predictors gives -in the logistic regression-the same result as the corresponding panel including $\kappa$ and $\lambda$ as predictors.

The panels indicated in italics would not be preferred due to the lower relative performance. All other panels show acceptable performance with AUC LOOCV>0.75 (for most panels AUC LOOCV >0.8).

While the invention has been illustrated and described in detail in the drawings and foregoing description, such illustration and description are to be considered illustrative or exemplary and not restrictive; the invention is not limited to the disclosed embodiments. For example, it is possible to present detection reagents for different biomarkers in different units.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain features of the invention are recited in mutually different dependent claims does not indicate that a combination of these features cannot be used to advantage. Any reference signs in the claims should not be construed as limiting the scope.

In sum, we hereby disclose an *in vitro* method for assessing whether a human patient is suffering from periodontitis. The method is based on the insight to determine biomarker proteins. Accordingly, in a sample of saliva from a patient, the concentrations are measured of the Free Light Chain $\kappa$ protein and/or the Free Light Chain $\lambda$ protein.

Based on the concentrations as measured, a value is determined reflecting the concentration or joint concentrations for said proteins. This value is compared with a threshold value reflecting in the same manner the concentration or joint concentrations associated with periodontitis. The comparison allows assessing whether the testing value is indicative of the presence of periodontitis in said patient. Thereby, typically, a testing value reflecting a concentration or joint concentration below the concentration or joint concentration reflected by the threshold value is indicative for the absence of periodontitis in said patient, and a testing value reflecting a concentration or joint concentration at or above the concentration or joint concentration reflected by the threshold value, is indicative for periodontitis in said patient.

## Claims

1. An *in vitro* method for assessing whether a human patient has periodontitis, wherein the method comprises:

   - detecting, in a sample of saliva from said human patient, the concentrations of the Free Light Chain κ protein and/or the Free Light Chain λ protein;
   - determining a testing value reflecting the concentration or joint concentrations determined for said protein or proteins;
   - comparing said testing value with a threshold value reflecting in the same manner the concentration or joint concentrations associated with periodontitis, so as to assess whether the testing value is indicative for periodontitis in said patient.

2. A method according to claim 1, wherein the human patient is suspected to have periodontitis; and/or wherein the age of the subject is determined and the testing value reflects the concentration or joint concentrations determined for said protein or proteins, in combination with the age of the subject.

3. A method according to any preceding claim, wherein the threshold value is based on the concentration or concentrations determined for the proteins in one or more reference samples each sample associated with the presence of periodontitis or absence of periodontitis.

4. A method according to claim 1 or claim 2, wherein the threshold value is based on the concentration or concentrations of the proteins in a set of samples, including samples from subjects that have periodontitis and samples from subjects not having periodontitis.

5. A method according to any one of the preceding claims, wherein the proteins consist of Free Light Chain κ protein and the Free Light Chain λ.

6. A method according to any one of claims 1 to 4, wherein the protein is the Free Light Chain λ.

7. A method according to any one of the preceding claims, wherein the concentration values determined are arithmetically processed into a number between 0 and 1.

8. The use of the proteins Free Light Chain κ protein and/or the Free Light Chain λ in a sample of saliva of a human patient, as biomarkers for assessing whether the patient has periodontitis, optionally wherein the age of the human patient is also used as a biomarker.

9. A system for assessing whether a human patient has periodontitis, the system comprising:

   - detection means able and adapted to detect in a sample of saliva of the human patient the Free Light Chain κ protein and/or the Free Light Chain λ;
   - a processor able and adapted to determine from the determined concentrations of said proteins an indication of the patient having periodontitis.

10. A system according to claim 9, further comprising a container for receiving an oral fluid sample, the container comprising the detection means.

11. A system according to claim 9 or 10, further comprising:

    - a user interface for presenting the indication to a user; and

- a data connection between the processor and the user interface for transferring the indication from the processor to the user interface.

12. A system according to any one of claims 9 to 11, wherein the processor is enabled to function by means of an internet-based application; and/or
wherein the interface is capable of putting in information on the age of the subject and the processor is able and adapted to determine from the determined concentration or concentrations, an indication that the patient has periodontitis.

13. An *in vitro* method for determining a change in status of periodontitis in a human patient suffering from periodontitis over a time interval from a first time point $t_1$ to a second time point $t_2$, the method comprising detecting, in at least one sample of saliva obtained from said patient at $t_1$ and in at least one sample of saliva obtained from said patient at $t_2$, the concentrations of the Free Light Chain $\kappa$ protein and/or the Free Light Chain $\lambda$, and comparing the concentrations, whereby a difference in any one or both of the concentrations, reflects a change in status.

14. An *in vitro* method of diagnosing whether a human patient has periodontitis, comprising detecting in a sample of saliva of the human patient the Free Light Chain $\kappa$ protein and/or the Free Light Chain $\lambda$, and assessing the presence of periodontitis in the patient on the basis of the concentration or concentrations of said proteins in said sample.

**Patentansprüche**

1. In-vitro-Verfahren zum Beurteilen, ob ein menschlicher Patient Parodontitis hat, wobei das Verfahren Folgendes umfasst:

   - Nachweisen, in einer Speichelprobe des menschlichen Patienten, der Konzentrationen des Proteins der freien Leichtkette $\kappa$ und/oder des Proteins der freien Leichtkette $\lambda$;
   - Bestimmen eines Testwerts, der die Konzentration oder die gemeinsamen Konzentrationen, die für das Protein oder die Proteine bestimmt werden, widerspiegelt;
   - Vergleichen des Testwerts mit einem Schwellenwert, der in gleicher Weise die Parodontitis zugeordnete Konzentration oder gemeinsamen Konzentrationen widerspiegelt, um zu beurteilen, ob der Testwert auf Parodontitis bei dem Patienten hinweist.

2. Verfahren nach Anspruch 1, wobei bei dem menschlichen Patienten der Verdacht besteht, dass er Parodontitis hat; und/oder
wobei das Alter des Subjekts bestimmt wird und der Testwert die Konzentration oder die gemeinsamen Konzentrationen, die für das Protein oder die Proteine bestimmt werden, in Kombination mit dem Alter des Subjekts widerspiegelt.

3. Verfahren nach einem vorstehenden Anspruch, wobei der Schwellenwert auf der Konzentration oder den Konzentrationen basiert, die für die Proteine in einer oder mehreren Referenzproben bestimmt werden, wobei jede Probe mit dem Vorhandensein von Parodontitis oder dem Fehlen von Parodontitis zugeordnet ist.

4. Verfahren nach Anspruch 1 oder Anspruch 2, wobei der Schwellenwert auf der Konzentration oder den Konzentrationen der Proteine in einem Satz von Proben basiert, der Proben von Subjekten, die Parodontitis haben, und Proben von Subjekten, die keine Parodontitis haben, einschließt.

5. Verfahren nach einem der vorstehenden Ansprüche, wobei die Proteine aus dem Protein der freien Leichtkette $\kappa$ und der freien Leichtkette $\lambda$ bestehen.

6. Verfahren nach einem der Ansprüche 1 bis 4, wobei das Protein die freie Leichtkette $\lambda$ ist.

7. Verfahren nach einem der vorstehenden Ansprüche, wobei die bestimmten Konzentrationswerte arithmetisch zu einer Zahl zwischen 0 und 1 verarbeitet werden.

8. Verwendung der Proteine der freien Leichtkette $\kappa$ und/oder der freien Leichtkette $\lambda$ in einer Speichelprobe eines menschlichen Patienten als Biomarker zum Beurteilen, ob der Patient Parodontitis hat, optional,
wobei das Alter des menschlichen Patienten auch als ein Biomarker verwendet wird.

9. System zum Beurteilen, ob ein menschlicher Patient Parodontitis hat, wobei das System umfasst:

- Nachweismittel, die in der Lage und angepasst sind, um in einer Speichelprobe des menschlichen Patienten das Protein der freien Leichtkette κ und/oder der freien Leichtkette λ nachzuweisen;
- einen Prozessor, der in der Lage und angepasst ist, um aus den bestimmten Konzentrationen der Proteine einen Hinweis darauf, dass der Patient Parodontitis hat, zu bestimmen.

10. System nach Anspruch 9, weiter umfassend einen Behälter zum Aufnehmen einer Mundflüssigkeitsprobe, wobei der Behälter die Nachweismittel umfasst.

11. System nach Anspruch 9 oder 10, weiter umfassend:

- eine Benutzerschnittstelle zum Darstellen des Hinweises für einen Benutzer; und
- eine Datenverbindung zwischen dem Prozessor und der Benutzerschnittstelle zum Übertragen des Hinweises von dem Prozessor an die Benutzerschnittstelle.

12. System nach einem der Ansprüche 9 bis 11, wobei der Prozessor dazu befähigt ist, mittels einer internetbasierten Anwendung zu arbeiten; und/oder
wobei die Schnittstelle in der Lage ist, Informationen über das Alter des Subjekts einzugeben, und der Prozessor in der Lage und angepasst ist, um aus der bestimmten Konzentration oder den bestimmten Konzentrationen einen Hinweis darauf, dass der Patient Parodontitis hat, zu bestimmen.

13. In-vitro-Verfahren zum Bestimmen einer Änderung im Status der Parodontitis bei einem menschlichen Patienten, der an Parodontitis leidet, über einen Zeitraum von einem ersten Zeitpunkt $t_1$ bis zu einem zweiten Zeitpunkt $t_2$, das Verfahren umfassend Nachweisen, in mindestens einer Speichelprobe, die von dem Patienten bei $t_1$ erhalten wird, und in mindestens einer Speichelprobe, die von dem Patienten bei $t_2$ erhalten wird, der Konzentrationen des Proteins der freien Leichtkette κ und/oder der freien Leichtkette λ, und Vergleichen der Konzentrationen, wobei eine Differenz in einer beliebigen oder beiden der Konzentrationen eine Änderung im Status widerspiegelt.

14. In-vitro-Verfahren zum Diagnostizieren, ob ein menschlicher Patient Parodontitis hat, umfassend Nachweisen, in einer Speichelprobe des menschlichen Patienten, des Proteins der freien Leichtkette κ und/oder der freien Leichtkette λ, und Beurteilen des Vorhandenseins von Parodontitis beim Patienten basierend auf der Konzentration oder den Konzentrationen der Proteine in der Probe.

**Revendications**

1. Procédé *in vitro* pour évaluer si un patient humain présente une parodontite, dans lequel le procédé comprend :

- la détection, dans un échantillon de salive dudit patient humain, des concentrations de la protéine de chaîne légère libre κ et/ou de la protéine de chaîne légère libre λ ;
- la détermination d'une valeur d'essai reflétant la concentration ou les concentrations communes déterminées pour ladite ou lesdites protéines ;
- la comparaison de ladite valeur d'essai à une valeur seuil reflétant de la même manière la concentration ou les concentrations communes associées à une parodontite, de manière à évaluer si la valeur d'essai indique une parodontite chez ledit patient.

2. Procédé selon la revendication 1, dans lequel le patient humain est suspecté de présenter une parodontite ; et/ou dans lequel l'âge du sujet est déterminé et la valeur d'essai reflète la concentration ou les concentrations communes déterminées pour ladite ou lesdites protéines, en association avec l'âge du sujet.

3. Procédé selon une quelconque revendication précédente, dans lequel la valeur seuil est basée sur la concentration ou les concentrations déterminées pour les protéines dans un ou plusieurs échantillons de référence, chaque échantillon étant associé à la présence d'une parodontite ou à l'absence d'une parodontite.

4. Procédé selon la revendication 1 ou la revendication 2, dans lequel la valeur seuil est basée sur la concentration ou les concentrations des protéines dans un ensemble d'échantillons, incluant des échantillons provenant de sujets qui présentent une parodontite et des échantillons provenant de sujets ne présentant pas de parodontite.

**5.** Procédé selon l'une quelconque des revendications précédentes, dans lequel les protéines consistent en la protéine de chaîne légère libre κ et de chaîne légère libre λ.

**6.** Procédé selon l'une quelconque des revendications 1 à 4, dans lequel la protéine est la chaîne légère libre λ.

**7.** Procédé selon l'une quelconque des revendications précédentes, dans lequel les valeurs de concentration déterminées sont traitées arithmétiquement en un nombre compris entre 0 et 1.

**8.** Utilisation des protéines de chaîne légère libre κ et/ou de chaîne légère libre λ dans un échantillon de salive d'un patient humain, comme biomarqueurs pour évaluer si le patient présente une parodontite, facultativement dans lequel l'âge du patient humain est également utilisé comme biomarqueur.

**9.** Système pour évaluer si un patient humain présente une parodontite, le système comprenant :

- des moyens de détection capables et adaptés pour détecter dans un échantillon de salive du patient humain la protéine de chaîne légère libre κ et/ou de chaîne légère libre λ ;
- un processeur capable et adapté pour déterminer, à partir des concentrations déterminées desdites protéines, une indication permettant de savoir si le patient présente une parodontite.

**10.** Système selon la revendication 9, comprenant en outre un récipient destiné à recevoir un échantillon de liquide buccal, le récipient comprenant les moyens de détection.

**11.** Système selon la revendication 9 ou 10, comprenant en outre :

- une interface utilisateur pour présenter l'indication à un utilisateur ; et
- une connexion de données entre le processeur et l'interface utilisateur pour transférer l'indication du processeur à l'interface utilisateur.

**12.** Système selon l'une quelconque des revendications 9 à 11, dans lequel le processeur est autorisé à fonctionner au moyen d'une application basée sur Internet ; et/ou dans lequel l'interface est capable d'introduire des informations sur l'âge du sujet et le processeur est capable et adapté pour déterminer, à partir de la concentration ou des concentrations déterminées, une indication permettant de savoir si le patient présente une parodontite.

**13.** Procédé *in vitro* pour déterminer un changement d'état de la parodontite chez un patient humain souffrant d'une parodontite sur un intervalle de temps allant d'un premier instant $t_1$ à un second instant $t_2$, le procédé comprenant la détection, dans au moins un échantillon de salive obtenu à partir dudit patient à $t_1$ et dans au moins un échantillon de salive obtenu à partir dudit patient à $t_2$, des concentrations de la protéine de chaîne légère libre κ et/ou de chaîne légère libre λ, et la comparaison des concentrations, selon lequel une différence dans l'une quelconque ou les deux concentrations reflète un changement d'état.

**14.** Procédé *in vitro* de diagnostic permettant de déterminer si un patient humain présente une parodontite, comprenant la détection dans un échantillon de salive du patient humain de la protéine de chaîne légère libre κ et/ou de chaîne légère libre λ, et l'évaluation de la présence de parodontite chez le patient sur la base de la concentration ou des concentrations desdites protéines dans ledit échantillon.

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014A037924 A

**Non-patent literature cited in the description**

- **RAMSEIER et al.** *J Periodontol.*, March 2009, vol. 80 (3), 436-46